# EUROPEAN PATENT SPECIFICATION

(11) **EP 0 705 824 B1**
(45) Date of publication and mention of the grant of the patent: **07.08.2002**
(21) Application number: 95916018.5
(22) Date of filing: 20.04.1995
(51) Int. Cl.: C07D 241/04

(54) **PROCESS FOR PRODUCING OPTICALLY ACTIVE N-TERT-BUTYL- 2-PIPERAZINE- CARBOXAMIDE**
VERFAHREN ZUR HERSTELLUNG VON OPTISCH AKTIVEM N-TERT-BUTYL-2-PIPERAZINCARBOXAMIDAMID
PROCEDE DE PRODUCTION DE N-TERT-BUTYL-2-PIPERAZINECARBOXAMIDE A ACTIVITE OPTIQUE

(30) Priority: 22.04.1994 JP 10772394; 25.01.1995 JP 3004495; 16.02.1995 JP 5334695
(43) Date of publication of application: 10.04.1996
(73) Proprietor: KOEI CHEMICAL CO., LTD., Osaka-shi, Osaka 541 (JP)
(72) Inventor: WATANABE, Nanao Koei Chemical Co., Ltd., Osaka-shi Osaka 536 (JP); TOKUDA, Yoko Koei Chemical Co., Ltd., Osaka-shi Osaka 536 (JP); KIKEGAWA, Kenichi Koei Chemical Co., Ltd., Osaka-shi Osaka 536 (JP); KURANISHI, Hideki Koei Chemical Co., Ltd., Osaka-shi Osaka 536 (JP); KURAHASHI, Takashi Koei Chemical Co., Ltd., Osaka-shi Osaka 536 (JP)
(74) Representative: VOSSIUS & PARTNER
(86) International application number: JP9500777
(87) International publication number: WO9529170

(56) References cited:
- WO-A-95/21162
- JP-A- 1 117 869
- JP-A- 3 279 375
- JP-A- 5 279 337
- JP-A- 49 110 830
- JP-A- 51 146 487
- M.ANSON: "ADVANCES IN PROTEIN CHEMISTRY VOL. IV" 1948 , ACADEMIC PRESS , NEW-YORK XP002006525 * page 349, paragraph 5B - page 352 *
- TETSUO SHIBA (STEREOCHEMISTRY) 1st EDIT., Kagakudojin Co. Ltd., 3rd line from the bottom, page 42 to line 1, page 43.
- TANEZO TAGUCHI (INTRODUCTION TO STEREOCHEMISTRY) REV. 2nd EDIT., Nanzando K.K., (1961), Line 10, page 88 to line 7, page 89.

## Description

The present invention relates to a process for the preparation of optically active N-tert.-butyl-2-piperazine carboxamide by optical resolution.

Optically active N-tert.-butyl-2-piperazine carboxamide is a useful compound as an intermediate to prepare medicines. For example, (S)-N-tert.-butyl-2-piperazine carboxamide is used as an intermediate to prepare N-(2(R)-hydroxy-1(S)-indanyl)-2(R)-phenylmethyl-4-(S)-hydroxy-5-(1-(4-(3-pyridylmethyl)-2(S)-N'-(tert.-butylcarboxamide)-piperazinyl))-pentaneamide which is a HIV protease inhibitor useful in the treatment of AIDS (cf. JP-A-5-279337). However, it is not known to prepare (S)-N-tert.-butyl-2-piperazine carboxamide by optical resolution of (R,S)-N-tert.-butyl-2-piperazine carboxamide.

JP-A-62-273433 describes that certain 2-piperazine carboxamide derivatives can be reduced with lithium aluminum hydride in a solvent.

JP-A-3-279375 discloses that an optically active 2-methylpiperazine is produced by reacting (±)-2-methylpiperazine with an optically active organic acid, e.g. L-tartaric acid, using water as the sole solvent, separating the produced two kinds of diastereomer salts from each other, taking advantage of the solubility difference of the salt to water, neutralizing the separated salt, and extracting with an organic solvent.

JP-A-62-273433 describes that certain 2-piperazine carboxamide derivatives can be reduced with lithium aluminum hydride in a solvent.

JP-A-3-279375 discloses that an optically active 2-methylpiperazine is produced by reacting (±)-2-methylpiperazine with an optically active organic acid, e.g. L-tartaric acid, using water as the sole solvent, separating the produced two kinds of diastereomer salts from each other, taking advantage of the solubility difference of the salt to water, neutralizing the separated salt, and extracting with an organic solvent.

US-A-3,912,738 relates to isomers and isomeric mixtures of N,N'-bis-[(1-formamido-2,2,2-trichloro)-ethyl]-piperazine and a method of isolating these isomers and isomeric mixtures from a conventionally prepared product.

EP-A-0541168 is concerned with compounds which inhibit the protease encoded by human immunodeficiency virus (HIV) or pharmaceutically acceptable salts thereof and are of value in the prevention of infection by HIV, the treatment of infection by HIV and the treatment of the resulting acquired immune deficiency syndrome (AIDS).

DE-A-2 625 258 discloses the conventional resolution of a racemic compound comprising a piperazinyl moiety.

US-A-3,912,738 relates to isomers and isomeric mixtures of N,N'-bis-[(1-formamido-2,2,2-trichloro)-ethyl]-piperazine and a method of isolating these isomers and isomeric mixtures from a conventionally prepared product.

EP-A-0541168 is concerned with compounds which inhibit the protease encoded by human immunodeficiency virus (HIV) or pharmaceutically acceptable salts thereof and are of value in the prevention of infection by HIV, the treatment of infection by HIV and the treatment of the resulting acquired immune deficiency syndrome (AIDS).

An object of the present invention is to provide a process for preparing optically active N-tert.-butyl-2-piperazine carboxamide having a high optical purity by optical resolution of (R,S)-N-tert.-butyl-2-piperazine carboxamide.

As a solution, of the above object it has been found that, when the optical resolution of (R,S)-N-tert.-butyl-2-piperazine carboxamide is carried out under certain conditions using an optically active lactic acid, as a resolving agent, optically active N-tert.-butyl-2-piperazine carboxamide having a high optical purity is easily obtained.

There is provided a process for preparing optically active N-tert.-butyl-2-piperazine carboxamide according to the claim comprising carrying out optical resolution of (R,S)-N-tert.-butyl-2-piperazine carboxamide using an optically active lactic acid as a resolving agent.

(R,S)-N-tert.-butyl-2-piperazine carboxamide which is used as a raw material in the present invention can be readily prepared by, for example, the following process:

First, 2-cyanopyrazine and tert.-butyl alcohol are reacted in the presence of sulfuric acid to obtain N-tert.-butyl-2-pyrazine carboxamide. Then, obtained N-tert.-butyl-2-pyrazine carboxamide is catalytically reduced with hydrogen in the presence of a Raney cobalt catalyst to obtain (R,S)-N-tert.-butyl-2-piperazine carboxamide which is a racemic mixture. Of course, (R,S)-N-tert.-butyl-2-piperazine carboxamide may be prepared by any other process.

(R,S)-N-tert.-butyl-2-piperazine carboxamide to be used in the present invention includes a mixture of equal amounts of the (R)-form and the (S)-form (namely, a racemic body), or a mixture containing one of the (R)-form and the (S)-form in a larger amount than the other.

As the resolving agent, optically active lactic acid is used.

The optically active lactic acid may be used in the L-form or the D-form, and either one of them is used to carry out the optical resolution. The amount of the optically active lactic acid is from 0.5 to 1.5 moles per one mole of (R,S)-N-tert.-butyl-2-piperazine carboxamide in the presence of a solvent comprising water wherein said solvent dissolves (R,S)-N-tert.-butyl-2-piperazine carboxamid and the optically active lactic acid and does not degrade them and in which one of the two diastereomer salts formed is precipitated therefrom as a less soluble salt.

In the optical resolution according to the present invention, a solvent is used generally. The solvent to be used is not limited, and any solvent can be used as long as (R,S)-N-tert.-butyl-2-piperazine carboxamide and the optically active lactic acid are both dissolved therein and are not degraded thereby, and one of the two diastereomer salts formed is precipitated therefrom as a less soluble salt. As such solvent, an acetone-water mixture, a methanol-water mixture, an acetone-methanol-water mixture are preferably used.

In one example of the optical resolution methods according to the present invention, (R,S)-N-tert.-butyl-2-piperazine carboxamide and the optically active lactic acid are dissolved in the solvent by heating it around 50°C while stirring. A resulting solution is concentrated by evaporation of the solvent and/or cooling, whereby among diastereomer salts formed from optically active N-tert.-butyl-2-piperazine carboxamide and the optically active lactic acid, a crystalline solid of a less soluble salt is precipitated. The precipitated crystalline solid is recovered by filtration, and recrystallized from the same solvent as used in the preparation of the above diastereomer salts to increase the optical purity of the diastereomer salt. From the filtrate from which the crystalline solid of the less soluble diastereomer salt has been separated, the other diastereomer salt can be recovered by concentration of the filtrate.

By salt decomposition of the separated diastereomer salt, free optically active N-tert.-butyl-2-piperazine carboxamide is obtained.

The salt decomposition can be effected by a per se conventional method. For example, to the obtained diastereomer salt, an aqueous solution containing an equivalent amount of an alkali such as a hydroxide of an alkali metal is added, and then water is evaporated off using an evaporator. After evaporation of water, to the residue, there is added a solvent, in which the alkali metal salt of the lactic acid formed by the reaction of the diastereomer salt with the alkali is substantially insoluble, and free optically active N-tert.-butyl-2-piperazine carboxamide is soluble, such as tetrahydrofuran. Then, a mixture is heated to about 50°C and mixed well. Thereafter, the alkali metal salt of the lactic acid is separated off by filtration. The filtrate is concentrated to dryness to obtain free optically active N-tert.-butyl-2-piperazine carboxamide.

When one of the enantiomers of N-tert.-butyl-2-piperazine carboxamide, which is useful as an intermediate for the preparation of medicines, is prepared by the optical resolution of (R,S)-N-tert.-butyl-2-piperazine carboxamide, the other enantiomer remains as a useless compound, so that the process becomes uneconomical.

When this useless enantiomer is racemized, and the obtained racemic mixture is again subjected to the optical resolution, the useful enantiomer of optically active N-tert.-butyl-2-piperazine carboxamide is obtained. By repeating the optical resolution and racemization, the useful enantiomer of optically active N-tert.-butyl-2-piperazine carboxamide can be prepared from (R,S)-N-tert.-butyl-2-piperazine carboxamide without leaving the useless enantiomer.

Optically active N-tert.-butyl-2-piperazine carboxamide is racemized in the presence of a basic compound.

As the basic compound alkali metal hydroxides, alkali metal alkoxides, alkali metal amides and alkali metal hydrides may be used. Examples of the alkali metal hydroxide are sodium hydroxide, potassium hydroxide, cesium hydroxide. Examples of the alkali metal alkoxide are sodium methoxide, sodium ethoxide, potassium methoxide, potassium ethoxide, potassium tert.-butoxide. Examples of the alkali metal amide are lithium diisopropyl amide, sodium amide. Examples of the alkali metal hydride are lithium hydride and sodium hydride.

The amount of the basic compound to be used is from 0.1 to 10 moles per one mole of optically active N-tert.-butyl-2-piperazine carboxamide. When the amount of the basic compound is less than this lower limit, the racemization takes a long time, while it is larger than this upper limit, the process is uneconomical.

As a solvent used in the racemization, one or a mixture of easily available solvents such as alcohols (e.g. methanol, ethanol), ethers (e.g. tetrahydrofuran), aromatic hydrocarbons (e.g. benzene, toluene, xylene), and acetonitrile is used.

The amount of the solvent is not limited. The amount of the solvent is usually from 0.5 to 10 wt. parts, preferably from 1 to 5 wt. parts per one wt. part of optically active N-tert.-butyl-2-piperazine carboxamide.

In one example of the racemization process, optically active N-tert.-butyl-2-piperazine carboxamide, the basic compound and the solvent are charged in a reactor and stirred at a temperature of 10 to 150°C, preferably 40 to 120°C for 1 to 20 hours, whereby optically active N-tert.-butyl-2-piperazine carboxamide is racemized.

Racemized (R,S)-N-tert.-butyl-2-piperazine carboxamide obtained can be isolated by a conventional method. For example, to a liquid which has been racemized, an acid such as hydrochloric acid is added to neutralize the basic compound. The neutralized liquid is concentrated to dryness with a rotary evaporator. Then, to the residue, a solvent such as acetonitrile or tetrahydrofuran is added, and the insoluble salt is removed by filtration. The filtrate is concentrated to precipitate (R,S)-N-tert.-butyl-2-piperazine carboxamide as a crystalline solid. Finally, the crystalline solid is isolated by filtration.

### Examples

In the following Examples, before the measurement of the optical purity, two nitrogen atoms in the piperazine ring of free N-tert.-butyl-2-piperazine carboxamide which was obtained by the salt decomposition of the diastereomer salt were benzoyled with benzoyl chloride. Then, the optical purity of the obtained dibenzoyl compound was measured by high performance liquid chromatography under the following conditions. The retention time of one optical enantiomer of the dibenzoyl compound (S-form) was 14.8 minutes, while that of the other enantiomer (R-form) was 17.5 minutes.

Analysis conditions in high performance liquid chromatography:
- Column:: SUMICHIRAL™ OA-4100
4.6 mm diameter x 250 mm
(manufactured by Sumitomo Chemical Co., Ltd.)
- Mobile phase:: Isopropanol/n-hexane (7/93 by volume)
(containing 0.5 % of acetic acid)
- Flow rate:: 1 ml/min.
- Detection:: UV 254 nm.

The total purity of the (S)-form and (R)-form of N-tert.-butyl-2-piperazine carboxamide was measured by gas chromatography using diethylene glycol mono-n-butyl ether as an internal standard under the following conditions:
Conditions in gas chromatographic analysis:
   - Column:: G-100 (manufactured by KAGAKUHIN KENSA KYOKAI)
   1.2 mm diameter x 40 m
   - Carrier gas:: helium at 25 ml/min.
   - Column temperature:: raising from 120°C to 230°C
   at a heating rate of 10°C/min.

### Reference Example 1

Racemic (R,S)-N-tert.-butyl-2-piperazine carboxamide (4.4 g, 24 mmol) and L-malic acid (3.2 g, 24 mmol) were added to a mixture of acetone (70 g) and water (34 g), and heated at 50°C while stirring. After keeping the same temperature for 30 minutes, the reaction mixture was cooled to room temperature, and kept standing overnight. The precipitated diastereomer salt was recovered by filtration and dried. Yield, 1.75 g (5.5 mmol).

To the salt, one mole per liter of an aqueous solution of sodium hydroxide containing twice moles of sodium hydroxide in relation to the diastereomer salt was added, and well mixed. Thereafter, water was distilled off by an evaporator. To the residue, tetrahydrofuran (21 g) was added, and the mixture was heated to 50°C and well mixed. Then, undissolved materials were separated by filtration at the same temperature, and the filtrate was concentrated to dryness to obtain optically active N-tert.-butyl-2-piperazine carboxamide having a specific rotatory power [α]²⁵_{D} of -19.6° (c = 3.8, methanol) (0.92 g, 5.0 mmol).

An optical purity of the (S)-form (retention time of the dibenzoyl compound: 14.8 minutes) was 80 %e.e.

### Example 1

Racemic (R,S)-N-tert.-butyl-2-piperazine carboxamide (10.0 g, 54.0 mmol) and a 85 % aqueous solution of L-lactic acid (5.73 g, 54.1 mmol) were added to a mixture of methanol (4 g) and acetone (20 g), and heated at 50°C while stirring. After keeping the same temperature for 30 minutes, the reaction mixture was gradually cooled to 10°C. The precipitated diastereomer salt was recovered by filtration, washed with a small amount of acetone, and dried. Yield, 5.10 g (18.5 mmol).

To the diastereomer salt, one mole per liter of an aqueous solution of sodium hydroxide containing the equimolar amount of sodium hydroxide to the diastereomer salt was added, and well mixed. Thereafter, the diastereomer salt was treated in the same manner as in Reference Example 1 to obtain (S)-N-tert.-butyl-2-piperazine carboxamide having a specific rotatory power [α]²⁵_{D} of -21.90° (c = 1.0, methanol) and an optical purity of 98.5 %e.e. (3.34 g, 18.0 mmol).

### Reference Exemple 2

(R)-N-tert.-butyl-2-piperazine carboxamide having a purity of 100 % and an optical purity of 100 %e.e. [a specific rotatory power [α]²⁵_{D} of +22.4° (c = 1.0, methanol)] (2.0 g) was added to toluene (10 g). To the mixture, a 20.7 wt. % solution of sodium methoxide in methanol (2.83 g) was added, and then kept at 80 to 82°C for 6 hours while stirring. After cooling to room temperature, the mixture was neutralized with hydrochloric acid, and concentrated to dryness with an evaporator under reduced pressure.

To the residue, acetonitrile (40 g) was added and maintained at 60°C for one hour, and then insoluble sodium chloride was removed by filtration. The filtrate was concentrated to dryness to obtain racemized N-tert.-butyl-2-piperazine carboxamide (2.0 g), which had a purity of 99.0 %, and an optical purity (of the R-form) of 0.2 %e.e and specific rotatory power [α]²⁵_{D} = +0.04° (c = 1.0, methanol).

### Reference Example 3

(R)-N-tert.-butyl-2-piperazine carboxamide having a purity of 100 % and an optical purity of 100 %e.e. [a specific rotatory power [α]²⁵_{D} of +22.4° (c = 1.0, methanol)] (2.0 g) was added to a 13.5 wt. % solution of sodium methoxide in methanol (20 g), and kept at 70°C for 16 hours while stirring. After cooling to room temperature, the mixture was treated in the same manner as in Example 3 to obtain racemized N-tert.-butyl-2-piperazine carboxamide (2.0 g), which had a purity of 98.0 %, and an optical purity (of the R-form) of 1.4 %e.e, and specific rotatory power [α]²⁵_{D} = +0.31° (c = 1.0, methanol).

### Reference Example 4

(R)-N-tert.-butyl-2-piperazine carboxamide having a purity of 100 % and an optical purity of 100 %e.e. [a specific rotatory power [α]²⁵_{D} of +22.4° (c = 1.0, methanol)] (2.0 g) was added to a 10.0 wt. % solution of potassium hydroxide in methanol (20 g), and kept at 70°C for 6 hours while stirring. After cooling to room temperature, the mixture was treated in the same manner as in Example 3 to obtain racemized N-tert.-butyl-2-piperazine carboxamide (2.0 g), which had a purity of 97.9 %, and an optical purity (of the R-form) of 65.0 %e.e, and specific rotatory power [α]²⁵_{D} = +14.55° (c = 1.0, methanol).

According to the present invention, optically active N-tert.-butyl-2-piperazine carboxamide having the high optical purity is easily prepared from (R,S)-N-tert.-butyl-2-piperazine carboxamide. In addition, optically active N-tert.-butyl-2-piperazine carboxamide which was readily racemized, can be used.

## Claims

1. A process for preparing optically active N-tert.-butyl-2-piperazine carboxamide comprising carrying out optical resolution of (R,S)-N-tert.-butyl-2-piperazine carboxamide using optically active lactic acid as a resolving agent, wherein the molar ratio of optically active lactic acid is from 0.5 to 1.5 moles to one mole of (R,S)-N-tert.-butyl-2-piperazine carboxamide, in the presence of a solvent comprising water wherein said solvent dissolves (R, S)-N-tert.-butyl-2-piperizine carboxamide and the optically active lactic acid and does not degrade them and in which one of the two diastereomer salts formed is precipitated therefrom as a less soluble salt.

## Patentansprüche

1. Ein Verfahren zur Herstellung von optisch aktivem N-tert-Butyl-2-piperazincarboxamid, umfassend Durchführen der optischen Trennung von (R,S)-N-tert.-Butyl-2-piperazincarboxamid unter Verwendung von optisch aktiver Milchsäure als einem Trennungsreagenz, wobei das molare Verhältnis von optisch aktiver Milchsäure von 0.5 bis 1.5 mol pro 1 mol (R,S)-N-tert.-Butyl-2-piperazincarboxamid beträgt, in Gegenwart eines Lösungsmittels, umfassend Wasser, wobei das Lösungsmittel (R,S)-N-tert.-Butyl-2-piperazincarboxamid und die optisch aktive Milchsäure löst und sie nicht abbaut und wobei eines der beiden gebildeten Diastereomerensalze als ein weniger lösliches Salz daraus ausfällt.

## Revendications

1. Procédé de fabrication de N-tert.-butyl-2-pipérazine carboxamide optiquement actif, comprenant l'opération consistant à effectuer la résolution optique du (R,S)-N-tert.-butyl-2-pipérazine carboxamide à l'aide d'acide lactique optiquement actif en tant qu'agent de résolution, dans lequel le rapport molaire de l'acide lactique optiquement actif est de 0,5 à 1,5 mole pour une mole de (R,S)-N-tert.-butyl-2-pipérazine carboxamide, en présence d'un solvant comprenant de l'eau, dans lequel ledit solvant dissout le (R,S)-N-tert.-butyl-2-pipérazine carboxamide et l'acide lactique optiquement actif et ne les dégrade pas et dans lequel l'un des deux sels diastéréoisomères formé est précipité à partir de celui-ci sous la forme d'un sel moins soluble.
